(19) **Europäisches Patentamt / European Patent Office / Office européen des brevets**

(11) **EP 4 722 347 A1**

(12) # EUROPEAN PATENT APPLICATION
published in accordance with Art. 153(4) EPC

(43) Date of publication:
**08.04.2026 Bulletin 2026/15**

(21) Application number: **24811138.7**

(22) Date of filing: **22.05.2024**

(51) International Patent Classification (IPC):
*C12N 5/0775* (2010.01)   *A61K 9/19* (2006.01)
*A61K 35/12* (2015.01)   *A61P 11/00* (2006.01)
*A61P 29/00* (2006.01)   *A61P 31/04* (2006.01)
*C12N 1/00* (2006.01)   *C12N 15/113* (2010.01)
*C12P 1/00* (2006.01)

(52) Cooperative Patent Classification (CPC):
**A61K 9/19; A61K 35/12; A61P 11/00; A61P 29/00; A61P 31/04; C12N 1/00; C12N 5/06; C12N 15/113; C12P 1/00**

(86) International application number:
**PCT/JP2024/018805**

(87) International publication number:
**WO 2024/242132 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 JP 2023085442**

(71) Applicant: **EXORPHIA, Inc.**
**Tokyo 100-0004 (JP)**

(72) Inventors:
• **IDE Izumi**
**Tokyo 100-0004 (JP)**
• **YONEKURA Toshiya**
**Tokyo 100-0004 (JP)**
• **SUMIYOSHI Issei**
**Tokyo 113-8421 (JP)**

(74) Representative: **Klöckner, Christoph**
**df-mp Patentanwälte Rechtsanwälte PartG mbB**
**Theatinerstraße 16**
**80333 München (DE)**

(54) **EXTRACELLULAR VESICLES OBTAINED FROM CELLS STIMULATED UNDER SPECIFIC CONDITIONS, AND PHARMACEUTICAL COMPOSITION CONTAINING SAID EXTRACELLULAR VESICLES**

(57)  The present disclosure provides an aqueous composition comprising extracellular vesicles. The aqueous composition of the present disclosure may be anti-inflammatory. The composition of the present disclosure may satisfy a certain quality standard for exerting an anti-inflammatory effect. The composition of the present disclosure can be preferably used in the treatment of inflammation.

**Description**

**TECHNICAL FIELD**

The present disclosure relates to extracellular vesicles obtained from cells stimulated under specific conditions, and a pharmaceutical composition comprising the said extracellular vesicles.

**BACKGROUND ART**

Exosomes are membrane-enclosed vesicles with a diameter of about 50 to 150 nm that are secreted from cells. Exosomes are considered to contain various biological molecules such as proteins, lipids, and nucleic acids, and are involved in various physiological functions such as intercellular communication, immune response, and cell differentiation.
Heat shock proteins (HSPs) are expressed when cells are exposed to stress (particularly heat stress). HSP70 has a role of increasing the secretion amount of exosomes or stabilizing the membrane of the said exosomes. Furthermore, heat shock is known to improve physiological functions such as the anti-inflammatory effect of mesenchymal stem cells (MSCs) (Non-Patent Literature 1). Accordingly, attempts are being made to prepare pharmaceuticals comprising cells subjected to heat shock.
Furthermore, Non-Patent Literature 1 discloses that mesenchymal stem cells subjected to heat shock under serum-free, albumin-free, platelet lysate-free, and growth factor-free conditions were further cultured under serum-free, albumin-free, platelet lysate-free, and growth factor-free conditions, and exosomes were recovered from the supernatant. Further, for example, Non-Patent Literature 2 discloses that cells were subjected to heat shock in a low glucose medium comprising fetal bovine serum (FBS) after exosome removal treatment, and then exosomes were obtained under 37°C, 5% $CO_2$ conditions. Non-Patent Literatures 3 and 4 disclose that mesenchymal stem cells cultured under normal medium conditions were further cultured under serum-free, albumin-free, platelet lysate-free, and growth factor-free conditions, and exosomes were recovered from the supernatant.

**PRIOR ART**

**NON-PATENT LITERATURES**

Non-Patent Literature 1: M. Yuce and E. Albayrak, 2022, Journal of Cellular Biochemistry, 132(2): 1966-1979
Non-Patent Literature 2: T. Yand et al., 2022, Journal of Biological Engineering, 16, Article number: 24
Non-Patent Literature 3: R. Kore et al., 2021, American Journal of Physiology-Regulatory, Integrative and Comparative Physiology, 321(5): R639-R654
Non-Patent Literature 4: L. Wang et al., 2022, Analytical Cellular Pathology, Article ID: 8708202

**SUMMARY OF THE INVENTION**

The present disclosure provides extracellular vesicles obtained from cells stimulated under specific conditions, and a pharmaceutical composition comprising the said extracellular vesicles. The present disclosure also provides a manufacturing method of the said extracellular vesicles.
According to the present disclosure, the following inventions may be provided.

(1A) An aqueous composition comprising extracellular vesicles, wherein:
the said extracellular vesicles

(i) express CD81, the expression amount of CD81 per extracellular vesicle is greater than or equal to a first reference value (or greater than or equal to a first reference magnification relative to the expression amount of CD81 in extracellular vesicles obtained by the same separation method from the same cell population under conditions (for example, in a second medium) where heat shock treatment is not performed and the treatment of changing the type of medium is not performed, and the first reference magnification is a magnification of 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times)), and,

the relative expression amount of CD81 to 1 g of CD63 is greater than or equal to a second reference value, the first reference value is a value of 12 (ag/particle) or more, and

the second reference value is a value of 50 (g/g CD63) or more, and,

(ii) the extracellular vesicles satisfy one or more of the following (ii-1) to (ii-2):

(ii-1) the extracellular vesicles express miR-4516, the expression amount thereof is greater than or equal to a second reference value, and the second reference value is a value of 500 amol/$10^9$ particles or more (for example, a value within the range of 500 amol/$10^9$ particles (preferably 2500 amol/$10^9$ particles) to 3900 amol/$10^9$ particles (or 500 amol/$10^9$ particles to 45,000 amol/$10^9$ particles)),

(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more (for example, 1.3 times to 2.2 times or about 1.3 times to about 26 times)), provided that, the second reference value is 1800 amol/$10^9$ particles or more, and the second reference magnification is 1.3 times or more.

Here, more preferably,
(1B) An aqueous composition comprising extracellular vesicles, wherein:
the extracellular vesicles

(i) express CD81, the expression amount of CD81 per extracellular vesicle is greater than or equal to a first reference value, and the first reference value is a value of 80 (ag/particle) or more, or the expression amount of CD81 is greater than or equal to a first reference magnification of the expression amount of CD81 in extracellular vesicles obtained by the same separation method from the same cell population under conditions (for example, in a second medium) where heat shock treatment is not performed and the treatment of changing the type of medium is not performed, and the first reference magnification is a magnification of 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times)), and,

(ii) the extracellular vesicles satisfy one or more of the following (ii-1) to (ii-2):

(ii-1) the extracellular vesicles express miR-4516, the expression amount thereof is greater than or equal to a second reference value, and the second reference value is a value of 500 amol/$10^9$ particles or more (for example, a value within the range of 500 amol/$10^9$ particles (preferably 2500 amol/$10^9$ particles) to 3900 amol/$10^9$ particles (or to 45,000 amol/$10^9$ particles)), and

(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more (for example, 1.3 times to 2.2 times (or about 1.3 times to about 26 times)).

Hereinafter, the said (1A) and the said (1B) are simply referred to as "the said (1)".
(2) The aqueous composition according to the said (1), wherein:
(ii-1) the extracellular vesicles express miR-4516, the expression amount thereof is greater than or equal to a second reference value, and the second reference value is a value of 500 amol/$10^9$ particles or more (for example, a value within the range of 500 amol/$10^9$ particles (preferably 2500 amol/$10^9$ particles) to 3900 amol/$10^9$ particles or to 45,000 amol/$10^9$ particles).
(3) The aqueous composition according to the said (1) or (2), wherein:
(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more (for example, 1.3 times to 2.2 times (or about 1.3 times to about 26 times)).
(4) The aqueous composition according to the said (1), wherein the extracellular vesicles satisfy (ii-1) and (ii-2).
(5) A lyophilized product of the composition according to any one of the said (1) to (4).
(6) A kit for extemporaneous preparation of an extracellular vesicle preparation, comprising the lyophilized product according to the said (5) and water for reconstitution.
(7) A method for manufacturing an aqueous composition comprising extracellular vesicles (for example, the said aqueous composition), comprising:

culturing mesenchymal stem cells under normal nutrient conditions;
subsequently, inducing a heat shock response in the said mesenchymal stem cells under serum-free, albumin-free, platelet lysate-free, and growth factor-free conditions;
further subsequently, culturing the mesenchymal stem cells in a medium that does not contain foreign exosomes and is suitable for cell proliferation, thereby secreting extracellular vesicles into the supernatant of the said medium, and recovering the supernatant comprising extracellular vesicles; and
separating the extracellular vesicles from the said supernatant to obtain an aqueous composition comprising extracellular vesicles.

(8) The method according to the said (7), further comprising filter sterilization.

(9) The composition according to any one of the said (1) to (4), the lyophilized product according to the said (5), or the kit according to the said (6), obtainable by the method according to the said (7) or (8).

(10) The composition according to any one of the said (1) to (4), the lyophilized product according to the said (5), or the kit according to the said (6), which is a pharmaceutical composition for use in treating inflammation in a subject.

(11) The composition, lyophilized product, or kit according to the said (9), wherein the inflammation is pneumonia.

(12) The composition, lyophilized product, or kit according to the said (10), wherein the inflammation is systemic inflammation.

(13) The composition, lyophilized product, or kit according to the said (10) or (12), wherein the inflammation is sepsis.

(14) The composition according to any one of the said (1) to (4), the lyophilized product according to the said (5), or the kit according to the said (6), for use in suppressing the expression of an adhesion molecule of vascular endothelial cells in a subject.

(15) The composition, lyophilized product, or kit according to the said (14), wherein the adhesion molecule is E-selectin.

(21) The method according to the said (7), wherein the first medium is serum-free, platelet lysate-free, albumin-free, and growth factor-free.

(22) The method according to the said (7), wherein the second medium is a serum-free medium and comprises one or more nutrients selected from the group consisting of growth factors, insulin, and transferrin.

(23) The method according to the said (22), wherein the second medium is foreign exosome-free.

(24) The method according to the said (21), wherein the second medium is a serum-free medium and comprises one or more nutrients selected from the group consisting of growth factors, insulin, and transferrin.

(25) The method according to the said (24), wherein the second medium does not comprise foreign exosome.

(31) The method according to any of the preceding, wherein the induction of the heat shock response is by maintaining the cells under a temperature condition of 38°C or higher.

(32) The method according to any of the preceding, wherein the induction of the heat shock response is by maintaining the cells under a temperature condition of 38.5°C or higher.

(33) The method according to any of the preceding, wherein the induction of the heat shock response is by maintaining the cells under a temperature condition of 39°C or higher.

(34) The method according to any of the preceding, wherein the induction of the heat shock response includes the enhancement of any one or more heat shock proteins.

(41) The method according to any of the preceding, wherein the separation of extracellular vesicles from the supernatant includes the steps of centrifugation, filtration, and/or size exclusion chromatography.

(42) The method according to the said (41), wherein the separation of extracellular vesicles from the supernatant includes the step of centrifugation.

(43) The method according to the said (41), wherein the separation of extracellular vesicles from the supernatant includes the step of filtration (for example, ultrafiltration).

(44) The method according to the said (41), wherein the separation of extracellular vesicles from the supernatant includes the step of size exclusion chromatography.

(51) The method according to any of the preceding, further comprising confirming one or more, or all expression selected from the group consisting of the expression CD81 expression and miR-4516 expression of the separated extracellular vesicles.

(52) The method according to the said (51), wherein the separated extracellular vesicles

(i) express CD81, the expression amount of CD81 per extracellular vesicle is greater than or equal to a first reference value,
the first reference value is a value of 80 (ag/particle) or more, or the expression amount of CD81 is 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times) of the expression amount of CD81 in extracellular vesicles obtained by the same separation method from the same cell population under conditions (for example, in a second medium) where heat shock treatment is not performed and the treatment of changing the type of medium is not performed, and/or,

(ii) the extracellular vesicles satisfy one or more selected from the group consisting of the following (ii-1) to (ii-4):

(ii-1) the extracellular vesicles express miR-4516, the expression amount thereof is greater than or equal to a second reference value, and the second reference value is a value of 500 amol/$10^9$ particles or more (for example, a value within the range of 500 amol/$10^9$ particles (preferably 2500 amol/$10^9$ particles) to 3900 amol/$10^9$ particles (or 500 amol/$10^9$ particles to 45,000 amol/$10^9$ particles)),

(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more.

(53) The method according to the said (51), which is performed so that the separated extracellular vesicles are:

(i) expressing CD81, the expression amount of CD81 per extracellular vesicle is greater than or equal to a first reference value,

the first reference value is a value of 80 (ag/particle) or more, or the expression amount of CD81 is greater than or equal to a first reference magnification of the expression amount of CD81 in extracellular vesicles obtained by the same separation method from the same cell population under conditions (for example, in a second medium) where heat shock treatment is not performed and the treatment of changing the type of medium is not performed, and the first reference magnification is a magnification of 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times), and/or,

(ii) the extracellular vesicles satisfy one or more selected from the group consisting of the following (ii-1) to (ii-4):

(ii-1) the extracellular vesicles express miR-4516, the expression amount thereof is greater than or equal to a second reference value, and the second reference value is a value of 500 amol/$10^9$ particles or more (for example, a value within the range of 500 amol/$10^9$ particles (preferably 2500 amol/$10^9$ particles) to 3900 amol/$10^9$ particles (or 500 amol/$10^9$ particles to 45,000 amol/$10^9$ particles)),

(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more.

(61) The composition, lyophilized product, or kit according to any of the preceding, for use in treating a subject who is likely to develop inflammation in a tissue, a subject who has inflammation in a tissue, and a subject who has fibrosis in a tissue.

(62) The composition, lyophilized product, or kit according to any of the preceding, for use in the increase of the M2/M1 macrophage ratio, prevention or treatment of inflammation, prevention or treatment of fibrosis of inflamed tissue, or suppression of adhesion molecule expression of blood vessels within inflamed tissue in a subject.

(63) The composition, lyophilized product, or kit according to the said (62), wherein the subject is a subject who is likely to develop inflammation in a tissue, a subject who has inflammation in a tissue, and a subject who has fibrosis in a tissue.

(71) The use of the aqueous composition according to any of the preceding in the manufacture of a composition, lyophilized product, or kit used for the application according to any of the preceding.

(81) A method for administering extracellular vesicles to a subject, comprising administering the composition, lyophilized product, or kit according to any of the preceding to the said subject.

(82) The method according to the said (81), wherein the subject is a subject who is likely to develop inflammation in a tissue, a subject who has inflammation in a tissue, and a subject who has fibrosis in a tissue.

(91) The aqueous composition, kit, or method according to any of the preceding, wherein the extracellular vesicles are CD63-positive and CD81-positive, and have a hydrodynamic diameter of 50 $\mu$m to 150 $\mu$m.

(92) The aqueous composition, kit, or method according to any of the preceding, wherein the extracellular vesicles are CD63-positive and CD81-positive.

(93) The aqueous composition, kit, or method according to any of the preceding, wherein the extracellular vesicles have a hydrodynamic diameter of 50 $\mu$m to 150 $\mu$m.

**BRIEF DESCRIPTION OF THE DRAWINGS**

[FIG. 1] FIG. 1 shows the particle size distribution of extracellular vesicles prepared from the supernatant obtained under Condition 5.

[FIG. 2] FIG. 2 shows the cytoprotective effect of extracellular vesicles each prepared from the supernatant obtained under any of Conditions 1 to 5 against silica-induced cell death in alveolar epithelial cells.

[FIG. 3] FIG. 3 shows the effect of extracellular vesicles each prepared from the supernatant obtained under any of Conditions 1 to 5 on the polarization of M0 macrophages to M1 macrophages (upper panel) and to M2 macrophages (lower panel).

[FIG. 4A] FIG. 4A shows the effect of extracellular vesicles each prepared from the supernatant obtained under Condition 1 or Condition 5 on the pneumonia tissue image in an Acute Respiratory Distress Syndrome (ARDS) model.

[FIG. 4B] FIG. 4B shows the effect of extracellular vesicles each prepared from the supernatant obtained under Condition 1 or Condition 5 on the lung injury score in an Acute Respiratory Distress Syndrome (ARDS) model.

[FIG. 5] FIG. 5 shows the effect of extracellular vesicles prepared from the supernatant obtained under Condition 5 on the number of leukocytes in the bronchoalveolar lavage fluid obtained from the ARDS model.

[FIG. 6] FIG. 6 shows the effect of extracellular vesicles prepared from the supernatant obtained under Condition 5 on the survival rate of the ARDS sepsis model.

[FIG. 7] FIG. 7 shows the effect of extracellular vesicles prepared from the supernatant obtained under Condition 5 on the E-selectin expression in vascular endothelial cells in the lung tissue of the ARDS model.

**DESCRIPTION OF THE EMBODIMENTS**

<Definitions>

In the present specification, the "subject" includes mammals, rodents such as mice and rats, domestic animals such as pigs and cows, pet animals such as dogs and cats, and primates such as humans and monkeys. The subject is preferably a human. The subject is a healthy individual, a subject at risk of developing a disease or condition (for example, inflammation or inflammatory disease), or a subject having a disease or condition (for example, inflammation or inflammatory disease).

In the present specification, "treatment" is used to include the meanings of therapy (therapeutic treatment) and prevention (prophylactic treatment). In the present specification, "therapy" means the therapy, cure, prevention, or improvement of remission of a disease or disorder, or the reduction of the rate of progression of a disease or disorder. In the present specification, "prevention" means reducing the possibility of the onset of a disease or condition, or delaying the onset of a disease or condition. Therapy may be performed on a subject having a disease or condition (for example, inflammation or inflammatory disease). Prevention may be performed on a healthy individual or a subject at risk of developing a disease or condition (for example, inflammation or inflammatory disease) (a subject before onset).

In the present specification, "therapeutically effective amount" means the amount of an agent that is effective for treating (preventing or treating) a disease or condition. A therapeutically effective amount of an agent can reduce the rate of worsening of the symptoms of a disease or condition, stop the worsening of the said symptoms, improve the said symptoms, cure the said symptoms, or suppress the onset or development of the said symptoms.

In the present specification, "effective amount" refers to the amount of an active ingredient that exhibits the expected effect.

In the present specification, "pharmaceutical composition" means a composition that exhibits a pharmacological action and has a composition suitable for administration to a subject. The pharmaceutical composition may include additives in addition to the pharmaceutically active ingredient (API). The pharmaceutical composition may be formulated, for example, for intravenous administration, intranasal administration, intratracheal administration, subcutaneous administration, intramuscular administration, or topical application, and may be administered intravenously, intranasally, intratracheally, subcutaneously, intramuscularly, or topically, respectively.

In the present specification, "mesenchymal stem cells" are a type of stem cell present in adults, and have the ability to differentiate into bone, cartilage, and fat (i.e., multipotency), which are mesodermal tissues. Mesenchymal stem cells can typically be obtained from tissues such as bone marrow, fat, dental pulp, menstrual blood, placenta, Wharton's jelly, umbilical cord, and umbilical cord blood. Human mesenchymal stem cells (human MSCs) can be identified by being adherent to plastic under standard culture conditions, being positive for cell surface markers CD73, CD90, and CD105, and being CD11b-negative or CD14-negative, CD19-negative or CD79α-negative, CD34-negative, and CD45-negative, and HLA-DR-negative.

In the present specification, "passage" means the operation of taking out a part or all of cultured cells from a culture vessel after culturing the cells in the culture vessel, and transferring them to a culture vessel containing a new medium. Passage is typically performed before the cells become confluent (a state where the cells completely cover the culture surface in the culture vessel). This is because when the cells become confluent, there is a risk that the properties of the cells will change due to dense contact between the cells, or the nutrients in the medium will be depleted, or the concentration of excretions from the cells into the medium will increase. In the present specification, the "number of passages" is indicated by the cumulative value of the number of subsequent passages, with the time when the cells are taken out from the living body being 0.

In the present specification, "extracellular vesicles" are vesicles secreted by cells. The membrane that the extracellular vesicles have is derived from the membrane that the cells have, and therefore contains at least one or more of the components on the cell membrane (for example, lipids and membrane proteins constituting the cell membrane). Extracellular vesicles include apoptotic vesicles (particle size 1 μm to 5 μm), microvesicles (particle size 100 nm to 1000 nm), and exosomes (particle size 50 nm to 150 nm). In vivo, cells secrete extracellular vesicles to the outside of the cell (particularly in body fluids), and under culture conditions, cells secrete extracellular vesicles to the outside of the cell (particularly in the culture medium). Extracellular vesicles are secreted from various cells, including mesenchymal stem cells (MSCs). Extracellular vesicles are typically obtained from cell culture supernatant or tissue supernatant by affinity chromatography, size exclusion chromatography, polymer precipitation, ultracentrifugation, and ultrafiltration, and combinations thereof. The properties of the extracellular vesicles obtained vary greatly depending on the acquisition method. Further, since cells secrete various extracellular vesicles to the outside of the cell, it is not always easy to identify, concentrate, enrich, isolate, or purify useful extracellular vesicles having specific properties from among them. Exosomes derived from mesenchymal stem cells may generally have anti-aging effects, whitening effects, skin regeneration effects, immunomodulation, anti-fibrotic effects, anti-inflammatory effects, anti-tumor effects, and therapeutic effects against neurodegenerative diseases (for example, Alzheimer's disease). Exosomes, depending on the cell of origin, typically express one or more, and typically all, selected from the group consisting of Alix, Tsg101, tetraspanins (CD81, CD63, and CD9), heat shock proteins (HSP60, HSP70, HSC70, and HSP90, etc.), and flotillin. Exosomes or the amount thereof can be confirmed, for example, by the expression of any one or more of the tetraspanins (preferably CD63 and CD81). Exosomes or the amount thereof can also be confirmed by particle size. Microvesicles (MVs) are formed by the outward budding of the cell membrane and have a diameter of about 100 nm to 1000 nm. MVs are known to express selectins, CD40, and the like. Apoptotic vesicles are vesicles produced by cells due to programmed cell death (apoptosis) and have a diameter of 50 nm to 5000 nm. Apoptotic vesicles are known to express Annexin V, phosphatidylserine, and the like.

In the present specification, "aqueous composition" means a composition comprising water as a solvent, or an aqueous solution. The aqueous composition has a composition suitable for maintaining the extracellular vesicles (for example, the composition of physiological saline). The aqueous composition, in one preferred embodiment, is a pharmaceutical composition.

In the present specification, "CD63" is a member of the tetraspanin protein family, has a transmembrane domain, and is present on the cell membrane and the exosome membrane. The standard sequence of human CD63 is registered as GenBank accession number AAH13017.1 in the National Center for Biotechnology Information (NCBI). CD63 includes a protein that has a sequence corresponding to that registered as GenBank accession number AAH13017.1 and has the function of CD63.

In the present specification, "CD81" is a member of the tetraspanin protein family, has a transmembrane domain, and is present on the cell membrane and the exosome membrane. The standard sequence of human CD81 is registered as GenBank accession number AAH93047.1 in the National Center for Biotechnology Information (NCBI). CD81 includes a protein that has a sequence corresponding to that registered as GenBank accession number AAH93047.1 and has the function of CD81.

In the present specification, "microRNA" (miRNA) is a type of non-coding RNA called microRNA. In the present specification, microRNA refers to mature miRNA (miR). MicroRNA is produced from precursor miRNA (also referred to as "precursor of miRNA") of about 70 bases in length. Precursor miRNA is produced in vivo by transcription from a gene encoding miRNA. Precursor miRNA is often processed in the cytoplasm to produce mature miRNA. Mature miRNA is typically a single-stranded RNA having a length of about 20-25 mer. MicroRNA regulates gene expression at the post-transcriptional level. MicroRNA is widely involved in various biological processes. Mature miRNA typically has a length of 20 to 25 bases. MiRNA is numbered in the order of identification and named miR-(number). When two mature miRNAs are produced from a precursor miRNA, the one with significantly higher expression is called miR-xx, and the one with significantly lower expression is called miR-xx* {where xx is a number and is numbered in the order of discovery}. However, if it is unclear which one is dominant, the suffix "-5p" is attached to the one arising from the 5' end of the precursor miRNA, and the suffix "-3p" is attached to the one arising from the 3' end. Also, human miR is named hsa-miR.

MiR-4516 is a type of microRNA. MiR-4516 is known to suppress renal fibrosis, etc. (Yoon et al., CELLs, 10(7):1682, 2021). MiR-4516 also has therapeutic significance in the treatment of psoriasis (Chowdhari et al., Biochim Biophys Acta Mol Basis Dis, 1863(12):3142-3152, 2017). MiR-4516 also suppresses the onset of retinitis (Pao et al., PloS One, 17(6): e0270526, 2022). Thus, miR-4516 is known to have anti-fibrotic effects and anti-inflammatory effects. The typical sequence of miR-4516 is encoded by the 2nd to 18th nucleotide sequence of the sequence registered as NCBI Reference Sequence NR_039741.1. MiR-4516 includes a microRNA that has a sequence corresponding to the 2nd to 18th nucleotide sequence of the sequence registered as NCBI Reference Sequence NR_039741.1 and has at least partially the function of miR-4516 (for example, anti-fibrotic effects and anti-inflammatory effects).

MiR-324-5p is a type of microRNA. MiR-324-5p suppresses apoptosis, inflammatory reaction, and oxidative stress after ischemia-reperfusion in the myocardium via TRAF3 (Jin et al., Int Immunopharmacol, 108:108740, 2022). MiR-324-5p has also been shown to reduce fibrotic lesions in a bleomycin-induced pulmonary fibrosis model mouse by the synthesis of a mimic molecule (Xu et al., Mol. Ther., 30(6):2370-2387, 2022). Thus, miR-324-5p is known to have anti-inflammatory effects and anti-fibrotic effects. The typical sequence of miR-324-5p is encoded by the 16th to 37th nucleotide sequence of the sequence registered as NCBI Reference Sequence NR_029896.1. MiR-324-5p includes a microRNA that has a sequence corresponding to the 16th to 37th nucleotide sequence of the sequence registered as NCBI Reference Sequence NR_029896.1 and has at least partially the function of miR-324-5p (for example, anti-fibrotic effects and anti-inflammatory effects).

In the present specification, "isolation" refers to the removal of at least one or more other components from the environment in which it was produced. Therefore, isolation does not require purification until it becomes a single component, and allows the presence of other components (for example, pharmaceutically acceptable contaminants) in the same composition. "Isolated microRNA" means microRNA in a free form. Free form is used to distinguish it from microRNA within vesicles or cells, and means microRNA outside vesicles or cells (including microRNA separated from vesicles or cells and artificially synthesized microRNA).

In the present specification, "inflammation" may be a phenomenon that appears for the purpose of a series of defenses to restore the damaged site when a cell or tissue is damaged. Inflammation can be induced by physical factors such as trauma, frostbite, burns, and radiation, chemical factors due to chemical substances, and immunological factors due to antibody reactions. Cells damaged by trauma secrete physiologically active substances such as inflammatory cytokines and chemokines, dilate blood vessels, increase vascular permeability, and induce antibodies, complement, plasma, and immune cells to the inflammatory site. "Inflammatory disease" is a systemic or local biological defense reaction to physical or chemical stimuli, stimuli by pathogenic bacteria, stimuli by components of pathogenic bacteria, stimuli by allergens, and the like. Inflammatory diseases include, for example, atopy, psoriasis, dermatitis, allergies, gingivitis, arthritis, pneumonia, rhinitis, otitis media, pharyngitis, tonsillitis, cystitis, nephritis, gastritis, hepatitis, pancreatitis, pelvic inflammation, vasculitis, inflammatory bowel disease (for example, intestinal lesions associated with Crohn's disease, Behçet's disease, ulcerative colitis, hemorrhagic proctitis, ileal pouchitis, ulcerative collitis), interstitial pneumonia, ankylosing spondylitis, influenza encephalopathy, systemic lupus erythematosus, endometriosis, acute respiratory distress syndrome, myocardial infarction, asthma, arteriosclerosis, edema, rheumatoid arthritis, delayed-type allergy (Type IV allergy), transplant rejection, graft-versus-host disease, autoimmune encephalomyelitis, multiple sclerosis, arthritis, cystic fibrosis, diabetic retinopathy, diabetic neuropathy, pressure ulcer, alopecia, ischemia-reperfusion injury, vascular restenosis, glomerulonephritis, and gastrointestinal allergy. An inflammatory disease may be an acute inflammatory disease or a chronic inflammatory disease (including acute exacerbation of a chronic inflammatory disease). An inflammatory disease may also be allergic. An inflammatory disease may also be necrotic.

In the present specification, "fibrosis" is the hardening or scarring of tissue caused by the excessive secretion and deposition of extracellular matrix proteins by fibroblasts in response to chronic inflammation. Fibrotic cellular responses are induced by harmful stimuli such as inflammation, toxins, infectious pathogens, autoimmune responses, and mechanical stress. Therefore, the treatment of inflammation leads to the treatment or prevention of fibrosis.

<Composition comprising Extracellular Vesicles of the Present Disclosure>

According to the present disclosure, a composition comprising extracellular vesicles (hereinafter referred to as the "composition of the present disclosure") is provided. In the composition of the present disclosure, the extracellular vesicles comprise CD81-positive (preferably CD63-positive and CD81-positive) extracellular vesicles (which may be extracellular vesicles known as exosomes).

In one embodiment, the extracellular vesicles express CD81, and the expression amount of CD81 per extracellular vesicle may be greater than or equal to a first reference value. In one embodiment, the expression amount of CD81 per extracellular vesicle is 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times) relative to the expression amount of CD81 in extracellular vesicles obtained by the same separation method from the same cell population under conditions (for example, in a second medium) where heat shock treatment is not performed and the treatment of changing the type of medium is not performed.

The first reference value (unit: ag/particle) may be a value of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, or 26 or more. The first reference value may be a value in the range of 14 to 26, 16 to 26, 18 to 26, or 20 to 26. Preferably, the expression amount of CD81 can be measured according to the product protocol of the CD81/CD81 Exosome ELISA Kit (Hakarel Co., Ltd., HAK-HEL8181-1). More preferably, the first reference value may be 78 or more, 79 or more, 80 or more, 85 or more, 90 or more, 95 or more, 100 or more, 105 or more, 110 or more, 115 or more, 120 or more, 125 or more, or 130 or more. The first reference value may also be 80 to 200, 100 to 170, 110 to 150, 120 to 140, or about 130. Preferably, the expression amount of CD81 can be measured by LC-MS/MS. Also, the expression amount of CD81 per extracellular vesicle may be a value of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, or 26 or more, and may be a value in the range of 14 to 26, 16 to 26, 18 to 26, or 20 to 26. Preferably, the expression amount of CD81 can be measured according to the product protocol of the CD81/CD81 Exosome ELISA Kit (Hakarel Co., Ltd., HAK-HEL8181-1), and more preferably, may be 78 or more, 79 or more, 80 or more, 85 or more, 90 or more, 95 or more, 100 or more, 105 or more, 110 or more, 115 or more, 120 or more, 125 or more, or 130 or more, and may be 80 to 200, 100 to 170, 110 to 150, 120 to 140, or about 130.

In one embodiment, the extracellular vesicles express miR-4516. In one preferred embodiment, the expression amount of miR-4516 is greater than or equal to a second reference value. Here, the second reference value (unit: $amol/10^9$ particles) may be a value of 500 or more, 600 or more, 700 or more, 800 or more, 900 or more, 1000 or more, 1100 or more, 1200 or more, 1300 or more, 1400 or more, 1500 or more, 1600 or more, 1700 or more, 1800 or more, 1900 or more, 2000 or more, 2100 or more, 2200 or more, 2300 or more, 2400 or more, 2500 or more, 2600 or more, 2700 or more, 2800 or more, 2900 or more, 3000 or more, 3100 or more, 3200 or more, 3300 or more, 3400 or more, 3500 or more, 3600 or more, 3700 or more, 3800 or more, or 3900 or more. The second reference value (unit: $amol/10^9$ particles) may be 45,000 or less, 40,000 or less, 35,000 or less, 30,000 or less, 25,000 or less, 20,000 or less, 15,000 or less, 10,000 or less, 9000 or less, 8000 or less, 7000 or less, 6000 or less, 5000 or less, 4000 or less, 3900 or less, 3800 or less, 3700 or less, 3600 or less, 3500 or less, 3400 or less, 3300 or less, 3200 or less, 3100 or less, 3000 or less, 2900 or less, 2800 or less, 2700 or less, 2600 or less, 2500 or less, 2400 or less, 2300 or less, 2200 or less, 2100 or less, 2000 or less, 1900 or less, or 1800 or less. In one preferred embodiment, the second reference value (unit: $amol/10^9$ particles) may be 1800 to 3900, and more preferably, may be 2300 to 3900 or 2300 to 45,000. In one embodiment, the second reference value (unit: $amol/10^9$ particles) may be in the numerical range of 2000 to 3500, 2500 to 3500, or 3000 to 3500. These reference values can be measured by quantitative real-time polymerase chain reaction (qRT-PCR).

In one embodiment, the extracellular vesicles express miR-4516. In one preferred embodiment, the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed. The second reference magnification may be a value of 1.1 times or more, 1.2 times or more, preferably 1.3 times or more, 1.4 times or more, more preferably 1.5 times or more, 1.6 times or more, 1.7 times or more, 1.8 times or more, 1.9 times or more, even more preferably 2 times or more, 2.1 times or more, or 2.2 times or more. The second reference magnification may also be a value of 26 times or less, 20 times or less, 15 times or less, 10 times or less, 9 times or less, 8 times or less, 7 times or less, 6 times or less, 5 times or less, 4 times or less, 3 times or less, 2.2 times or less, 2.1 times or less, 2 times or less, 1.9 times or less, 1.8 times or less, 1.7 times or less, 1.6 times or less, 1.5 times or less, 1.4 times or less, 1.3 times or less, 1.2 times or less, or 1.1 times or less. The second reference magnification may be a value in the range of, for example, 1.1 times to 2.2 times, preferably 1.3 times to 2.2 times, more preferably 1.5 times to 2.2 times, and even more preferably 2 times to 2.2 times.

In one embodiment, the extracellular vesicles express miR-324-5p. In one preferred embodiment, the expression amount of miR-324-5p is greater than or equal to a third reference value. Here, the third reference value (unit: $amol/10^9$ particles) may be a numerical value of 0.1 or more, 0.2 or more, 0.25 or more, 0.3 or more, 0.4 or more, 0.5 or more, 0.6 or more, 0.7 or more, 0.8 or more, or 0.85 or more. The third reference value (unit: $amol/10^9$ particles) may also be a numerical value of 0.85 or less, 0.8 or less, 0.7 or less, 0.6 or less, 0.5 or less, 0.4 or less, 0.3 or less, or 0.25 or less. In one preferred embodiment, the third reference value (unit: $amol/10^9$ particles) may be 0.25 to 0.85, and more preferably, may be 0.6 to 0.85.

In one embodiment, the extracellular vesicles express miR-324-5p. In one preferred embodiment, the expression amount of miR-324-5p is a value greater than or equal to a third reference magnification relative to the expression amount of miR-324-5p in extracellular vesicles obtained by the same separation method from the same cell population under conditions (for example, in a second medium) where heat shock treatment is not performed and the treatment of changing the type of medium is not performed. The third reference magnification may be a value of 1.1 times or more, 1.2 times or more, preferably 1.3 times or more, 1.4 times or more, more preferably 1.5 times or more, 1.6 times or more, 1.7 times or more, 1.8 times or more, 1.9 times or more, even more preferably 2 times or more, 2.5 times or more, even more preferably 2.7 times or more, 3 times or more, 3.5 times or less, or 4 times or more. The third reference magnification may also be a value of 4 times or less, 3.5 times or less, 3 times or less, 2.5 times or less, 2 times or less, 1.9 times or less, 1.8 times or less, 1.7 times or less, 1.6 times or less, 1.5 times or less, 1.4 times or less, 1.3 times or less, 1.2 times or less, or 1.1 times or less. The second reference magnification may be a value in the range of, for example, 1.1 times to 4 times, preferably 2 times to 4 times, more preferably 2.5 times to 4 times, and even more preferably 3 times to 4 times.

In one preferred embodiment, the extracellular vesicles satisfy one or more selected from the group consisting of the following (ii-1) to (ii-2):

(ii-1) the extracellular vesicles express miR-4516, the expression amount thereof is greater than or equal to a second reference value, and the second reference value is a value within the range of 500 amol/$10^9$ particles to 3900 amol/$10^9$ particles (or 500 amol/$10^9$ particles to 45,000 amol/$10^9$ particles), and

(ii-2) the expression amount of the miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more.

In one preferred embodiment, the extracellular vesicles satisfy one or more selected from the group consisting of the following (ii-1) to (ii-4):

(ii-1) the extracellular vesicles express miR-4516, the expression amount thereof is greater than or equal to a second reference value, and the second reference value is a value within the range of 500 amol/$10^9$ particles to 3900 amol/$10^9$ particles (or 500 amol/$10^9$ particles to 45,000 amol/$10^9$ particles), (ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more,

(ii-3) the extracellular vesicles express miR-324-5p, the expression amount thereof is greater than or equal to a third reference value, and the third reference value is a value within the range of 0.1 amol/$10^9$ particles to 0.89 amol/$10^9$ particles, and,

(ii-4) the expression amount of miR-324-5p is a value greater than or equal to a third reference magnification relative to the expression amount of miR-324-5p in extracellular vesicles obtained by the same separation method from the same cell population under conditions (for example, in a second medium) where heat shock treatment is not performed and the treatment of changing the type of medium is not performed, and the third reference magnification is a value of 1.1 times or more (preferably 1.3 times or more), provided that, the second reference value is 1800 amol/$10^9$ particles or more, the third reference value is 0.25 amol/$10^9$ particles or more, and the second reference magnification is 1.3 times or more.

In one preferred embodiment, the extracellular vesicles satisfy all of the following (ii-1) and (ii-3):

(ii-1) the extracellular vesicles express miR-4516, the expression amount thereof is greater than or equal to a second reference value, and the second reference value is a value within the range of 500 amol/$10^9$ particles to 3900 amol/$10^9$ particles (or 500 amol/$10^9$ particles to 45,000 amol/$10^9$ particles), and

(ii-3) the extracellular vesicles express miR-324-5p, the expression amount thereof is greater than or equal to a third reference value, and the third reference value is a value within the range of 0.1 amol/$10^9$ particles to 0.89 amol/$10^9$ particles, provided that, the second reference value is 1800 amol/$10^9$ particles or more, or the third reference value is 0.25 amol/$10^9$ particles or more.

In one preferred embodiment, the extracellular vesicles satisfy one or more selected from the group consisting of the following (ii-2) and (ii-4):

(ii-2) the expression amount of the miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more, and

(ii-4) the expression amount of miR-324-5p is a value greater than or equal to a third reference magnification relative to the expression amount of miR-324-5p in extracellular vesicles obtained by the same separation method from the same cell population under conditions (for example, in a second medium) where heat shock treatment is not performed and the treatment of changing the type of medium is not performed, and the third reference magnification is a value of 1.1 times or more, provided that, the second reference magnification is 1.3 times or more, or the third reference magnification is 2.7 times or more.

The extracellular vesicles of the present disclosure can control the M1/M2 polarization of macrophages in tissue. In one preferred embodiment, the extracellular vesicles of the present disclosure can reduce M1 macrophages and/or increase M2 macrophages in tissue. Therefore, the composition comprising the extracellular vesicles of the present disclosure can be used for reducing M1 macrophages and/or increasing M2 macrophages in tissue. In one preferred embodiment, the extracellular vesicles of the present disclosure have an anti-inflammatory effect. The anti-inflammatory effect can be exerted locally or systemically. The chronicity of inflammation induces fibrosis of the tissue. Therefore, the anti-inflammatory effect serves as the prevention of fibrosis of the tissue.

The extracellular vesicles of the present disclosure can reduce the expression of adhesion molecules in vascular endothelial cells. The adhesion molecule is preferably E-selectin.

<Method for Manufacturing the Composition of the Present Disclosure>

The extracellular vesicles in the composition of the present disclosure are usually secreted from cells, and are preferably secreted from multipotent stromal cells (particularly mesenchymal stem cells). Therefore, the composition of the present disclosure can be obtained as a secretion product of the said cells.

The mesenchymal stem cells include bone marrow-derived mesenchymal stem cells, fat-derived mesenchymal stem cells, dental pulp-derived mesenchymal stem cells, umbilical cord-derived mesenchymal stem cells, umbilical cord blood-derived mesenchymal stem cells, Wharton's jelly-derived mesenchymal stem cells, placenta-derived mesenchymal stem cells, menstrual blood-derived mesenchymal stem cells, and dental pulp-derived mesenchymal stem cells, all of which can be preferably used as a source of extracellular vesicles. In one embodiment, the mesenchymal stem cells are any one or more selected from the group consisting of bone marrow-derived mesenchymal stem cells, fat-derived mesenchymal stem cells, and umbilical cord-derived mesenchymal stem cells, and may be, for example, bone marrow-derived mesenchymal stem cells or umbilical cord-derived mesenchymal stem cells.

It is general to culture cells in a medium (for example, a nutritious medium) under conditions of 37°C and 5% $CO_2$, and after culturing, the supernatant containing extracellular vesicles is recovered. When extracellular vesicles are utilized as a pharmaceutically active ingredient, the medium is often completely replaced with a chemically defined medium after culturing for the purpose of preventing the mixing of animal components and the like into the supernatant, and then the supernatant containing extracellular vesicles released into the said medium is recovered.

A basal medium can be used as the medium. The basal medium includes Dulbecco's Modified Eagle Medium (DMEM), αMEM, Minimum Essential Medium (MEM), Knockout DMEM (KO-DMEM), Glasgow Minimum Essential Medium (G-MEM), Eagle's Basal Medium (BME), DMEM/Ham's F12, Advanced DMEM/Ham's F12, Iscove's Modified Dulbecco's Medium, Ham's F-10, Ham's F-12, Medium 199, RPMI1640 Medium, Williams' Medium E, MCDB Medium, Fisher's Medium, and combinations and/or modifications thereof.

In one embodiment, the medium, in addition to the basal medium, further comprises sugars such as glucose, amino acids (for example, glutamine), transferrin, cytokines, lipids, growth factors (for example, basic fibroblast growth factor (bFGF), transforming growth factor (TGF) β3, platelet-derived growth factor (PDGFbb)), antibiotics, antifungal agents, steroid hormones, protein hormones (for example, insulin), trace metals (for example, selenium), or combinations thereof. For adherent culturing of cells, it is preferable to culture them on a culture surface having cell adhesiveness. Culture vessels having such a culture surface are commercially available and can be used as appropriate. The medium is buffered with a pH buffering agent and has a neutral pH (for example, typically pH 6.8 to 7.4, for example, pH 7.2 to 7.4).

The method for producing the composition of the present disclosure may include the following steps:

a step of performing heat shock treatment on cells in a first medium;
a step of culturing the heat-shock-treated cells in a second medium to obtain a culture comprising extracellular vesicles in the supernatant; and
a step of recovering the supernatant comprising extracellular vesicles from the culture.

In one embodiment, the first medium is a serum-free medium. The first medium is preferably one that does not apply stress or stimulation to the cells, which makes it possible to apply only heat shock stress to the cells, and the effect of heat shock stress can remain stable. In one preferred embodiment, the first medium is a platelet lysate-free, serum-free, and albumin-free medium. In one preferred embodiment, the first medium may be a platelet lysate-free, serum-free, albumin-free, and growth factor-free medium. In one specific embodiment, the first medium is platelet lysate-free, serum-free, albumin-free, and growth factor-free, and comprises neither transferrin nor insulin. Here, the first medium may or may not comprise glucose, but preferably does not comprise glucose. In one embodiment, the first medium is a starvation medium. In one preferred embodiment, the first medium is suitable for short-term maintenance of cells (for example, 1 to 4 hours). In one preferred embodiment, the first medium has the composition of a basal medium. In one preferred embodiment, the first medium has the composition of a basal medium comprising an additive, and the additive is glucose.

In one embodiment, the second medium is a serum-free medium. In one embodiment, the second medium is a medium suitable for cell proliferation. In one preferred embodiment, the second medium is a platelet lysate-free and serum-free medium that is suitable for cell proliferation, from the viewpoint of preventing the mixing of exosomes from the medium. A medium suitable for cell proliferation contains an effective amount of nutrients required for cell proliferation and can proliferate cells in the said medium. That is, the second medium proliferates cells more strongly compared to the first medium. In one preferred embodiment, the second medium may be a nutritious medium. In one preferred embodiment, the second medium is platelet lysate-free and serum-free, and comprises albumin (preferably serum albumin, particularly preferably human serum albumin). In one preferred embodiment, the second medium is a medium free of foreign extracellular vesicles (particularly exosomes), from the viewpoint of preventing the mixing of foreign extracellular vesicles into the culture supernatant. It is generally preferable to use a medium free of foreign extracellular vesicles as the second medium, unless foreign extracellular vesicles are intentionally mixed. Foreign extracellular vesicles are all extracellular vesicles other than the extracellular vesicles released from the cells being cultured. Since platelet lysate and serum contain foreign extracellular vesicles, it is preferable not to mix them into the second medium. Usually, platelet lysate and serum from which extracellular vesicles have been removed may also contain residual extracellular vesicles, and it is preferable not to mix platelet lysate and serum from which extracellular vesicles have been removed into the medium. In one preferred embodiment, the second medium further comprises a growth factor, and more preferably, further comprises transferrin and insulin. In a preferred embodiment, the second medium has a different composition from the first medium. In one embodiment, the second medium is a recovery medium. In one embodiment, the second medium, in addition to the composition of the first medium, further comprises one or more selected from the group consisting of albumin, transferrin, insulin, and growth factors. The growth factors may comprise one, two, or all selected from the group consisting of basic fibroblast growth factor (bFGF), transforming growth factor (TGF, for example, TGF-β3), and platelet-derived growth factor (PDGF, for example, PDGFbb). The culturing in the second medium can preferably be performed for a time sufficient for cell proliferation. The culturing in the second medium can be performed, for example, at 37°C for, for example, 24 to 96 hours, 24 to 36 hours, 24 to 48 hours, or 48 to 96 hours.

The term "medium" as used in the present specification contains sufficient nutrients for cell proliferation and maintenance by the medium alone. The medium (for example, nutritious medium) may be a platelet lysate-containing medium, serum-containing medium, albumin-containing medium, platelet lysate-free medium, serum-free medium, serum-free albumin-free medium, xeno-free medium, or chemically defined medium. A chemically defined medium means a medium in which all components are known. A chemically defined medium may be a completely synthetic medium. Since platelet lysate and serum may be contaminated with extracellular vesicles, it is desirable to avoid such contamination for the purpose of recovering extracellular vesicles from the culture. From this viewpoint, the medium (particularly the nutritious medium) may preferably be a platelet lysate-free medium, serum-free medium, serum-free albumin-free medium, xeno-free medium, or chemically defined medium. The medium may not comprise a growth factor or a proliferation factor, but may comprise a growth factor and/or a proliferation factor.

The term "starvation medium" as used in the present specification is a serum-free, albumin-free, and growth factor-free medium. The starvation medium typically substantially does not comprise factors that induce the activation of specific signal transduction pathways to direct cell proliferation and differentiation. For example, the starvation medium may not comprise factors necessary for cell proliferation such as glucose and L-glutamine. The starvation medium may be, for example, buffered physiological saline or a buffered basal medium. However, the starvation medium may comprise nutrients (for example, glucose, etc.) necessary for culturing cells for 1 to 5 days, for example, 1 day, 2 days, or 3 to 5 days. The starvation medium is preferably a chemically defined medium. The starvation medium, in one embodiment, comprises a basal medium and glucose.

The term "recovery medium" as used in the present specification is a serum-free medium and is a medium comprising nutrients necessary for cell culturing. The recovery medium can use the medium defined in the said "medium", and may be, for example, a medium comprising sufficient nutrients for cell proliferation and maintenance, and is, for example, serum-free. The recovery medium preferably comprises human serum albumin (HSA). The recovery medium is serum-free and preferably comprises HSA and a growth factor. The growth factor may be any one or more, or all selected from the group consisting of bFGF, TGF-β, and PDGF, for example, the growth factors exemplified above. The recovery medium may preferably comprise HSA, a growth factor, insulin, and transferrin. In one preferred embodiment, the recovery medium may be a nutritious medium that provides preferred proliferation conditions exceeding a normal medium.

Heat shock is a treatment that induces a heat shock response in cells. Heat shock can be applied to cells by maintaining the cells, for example, under a temperature condition of 38°C or higher, 38.5°C or higher, 39°C or higher, preferably 40°C or higher, and particularly about 42°C. The maintenance time may be, for example, 1 hour or more, preferably 1 hour to 2 hours. The heat shock response includes the enhancement (for example, activation or increased expression) of any one or more heat shock proteins (for example, Hsp70, Hsc70, Hsp90, Hsp32, and Hsp27, etc.). When heat shock is applied to cells, the expression level of heat shock protein usually increases in the said cells. The expression level can be quantified by methods such as Western blotting, ELISA, and immunofluorescence staining. The mRNA expression of heat shock protein can be quantified by methods such as quantitative real-time PCR. Heat shock can be performed under conditions where the majority of cells (for example, about 65% to about 99%, particularly about 70% to about 98%) can survive.

Extracellular vesicles can be isolated or purified from the supernatant comprising extracellular vesicles. Isolation or purification can be performed by centrifugation, filtration, and/or size exclusion chromatography. Extracellular vesicles in which the abundance of each of the said microRNAs in the obtained extracellular vesicles (particularly exosomes) is greater than or equal to the reference value may be used as the pharmaceutically active ingredient. The quality of the extracellular vesicles obtained may vary depending on the purification method. Therefore, the quality control of the extracellular vesicles used as the active ingredient may be performed by sampling a part of the obtained extracellular vesicles, quantifying each of the said microRNAs, and comparing it with the reference value.

In a preferred embodiment of the present disclosure, the pharmaceutical composition may comprise extracellular vesicles at a concentration of $1 \times 10^8$/mL or more, $2 \times 10^8$/mL or more, $3 \times 10^8$/mL or more, $4 \times 10^8$/mL or more, $5 \times 10^8$/mL or more, $6 \times 10^8$/mL or more, $7 \times 10^8$/mL or more, $8 \times 10^8$/mL or more, $9 \times 10^8$/mL or more, $1 \times 10^9$/mL or more, $2 \times 10^9$/mL or more, $3 \times 10^9$/mL or more, $4 \times 10^9$/mL or more, $5 \times 10^9$/mL or more, $6 \times 10^9$/mL or more, $7 \times 10^9$/mL or more, $8 \times 10^9$/mL or more, $9 \times 10^9$/mL or more, $1 \times 10^{10}$/mL or more, $2 \times 10^{10}$/mL or more, $3 \times 10^{10}$/mL or more, $4 \times 10^{10}$/mL or more, $5 \times 10^{10}$/mL or more, $6 \times 10^{10}$/mL or more, or $7 \times 10^{10}$/mL or more. The number of extracellular vesicles can be determined by the nanoparticle tracking analysis method using ZetaView (Particle Metrix).

The concentrated extracellular vesicles may have, for example, a D10 of 100 or less, a D50 of 160 or less, and a D90 of 280 or less. The extracellular vesicles may have, for example, a D10 of 90 or less, 88 or less, 86 or less, 84 or less, 82 or less, 80 or less, 78 or less, 76 or less, 74 or less, 72 or less, or 70 or less, a D50 of 150 or less, 140 or less, 130 or less, 120 or less, or 110 or less, and a D90 of 250 or less, 240 or less, 230 or less, 220 or less, 210 or less, 200 or less, 190 or less, or 180 or less. D10 means the particle size at which 10% of the particles have a particle size less than or equal to this particle size, D50 means the median particle size, and D90 means the particle size at which 90% of the particles have a particle size less than or equal to this particle size among the entire particles. The particle size concerning the said particle size distribution (for example, D10, D50, and D90) can be determined by the nanoparticle tracking analysis method using ZetaView (Particle Metrix). The amount of exosomes contained in the extracellular vesicles can be determined based on the expression of CD81, and more preferably, based on the expression of CD63 and CD81. The quantification of protein may be performed by ELISA, but can preferably be determined by mass spectrometry. Mass spectrometry can be performed by LC-MS/MS. Mass spectrometry requires an internal standard. The internal standard may be a partial peptide of the protein.

The pharmaceutical composition of the present disclosure may further comprise additives in addition to extracellular vesicles and an aqueous solvent. The aqueous solvent includes water. The additives include salts, isotonic agents, pH buffering agents, and the like. The pharmaceutical composition of the present disclosure is formulated for oral administration and parenteral administration. The parenteral administration is not particularly limited, but examples include intravenous administration, intranasal administration, intratracheal administration, intraperitoneal administration, intramuscular administration, intradermal administration, subcutaneous administration, intraventricular administration, intracerebrospinal administration, intraportal administration, intracardiac administration, and rectal administration (enema). The parenteral administration may also be an injection of the pharmaceutical composition into a lesion or affected site.

The pharmaceutical composition of the present disclosure may be a lyophilized preparation. The lyophilized preparation may comprise a lyophilized product of the pharmaceutical composition of the present disclosure. The lyophilized preparation may preferably be a combination preparation of the lyophilized product of the pharmaceutical composition of the present disclosure and water for reconstitution. The said combination preparation may be a kit for the preparation of an extracellular vesicle preparation, and may be a kit for extemporaneous preparation of an extracellular vesicle preparation. Medical professionals such as physicians can prepare an extracellular vesicle preparation by reconstituting the lyophilized product of the pharmaceutical composition of the present disclosure with water for reconstitution before administration.

<Applications of the Composition of the Present Disclosure>

The composition of the present disclosure can be used for various applications. Such applications may be the increase of the M2/M1 macrophage ratio, prevention or treatment of inflammation, prevention or treatment of fibrosis of inflamed tissue, or suppression of adhesion molecule expression of blood vessels within inflamed tissue. The composition of the present disclosure can also be used for treating a subject who is likely to develop inflammation in a tissue, a subject who has inflammation in a tissue, and a subject who has fibrosis in a tissue.

In one embodiment, the composition of the present disclosure can be used for the increase of the M2/M1 macrophage ratio, prevention or treatment of inflammation, prevention or treatment of fibrosis of inflamed tissue, or suppression of adhesion molecule expression of blood vessels within inflamed tissue in a subject. The subject may be a subject who is likely to develop inflammation in a tissue, a subject who has inflammation in a tissue, a subject who has fibrosis in a tissue, or a subject who has increased expression of vascular adhesion molecules. That is, in one embodiment, the composition of the present disclosure can be used for the increase of the M2/M1 macrophage ratio, prevention or treatment of inflammation, prevention or treatment of fibrosis of inflamed tissue, or suppression of adhesion molecule expression of blood vessels within inflamed tissue in a subject who is likely to develop inflammation in a tissue, a subject who has inflammation in a tissue, a subject who has fibrosis in a tissue, or a subject who has increased expression of vascular adhesion molecules. The adhesion molecule may be, for example, E-selectin. The increase of the M2/M1 macrophage ratio may be an increase compared to the M2/M1 macrophage ratio in the same tissue of a healthy person, or an increase of the pathological said ratio.

In one embodiment, the composition of the present disclosure can be administered to a subject having a tissue with a reduced M2/M1 macrophage ratio compared to the M2/M1 macrophage ratio in a non-inflamed tissue. The composition of the present disclosure can improve the reduced M2/M1 macrophage ratio (that is, improve the M2/M1 macrophage ratio, or bring it closer to or equal to the ratio in a non-inflamed tissue).

According to the present disclosure, the use of the extracellular vesicles of the present disclosure in the manufacture of a medicament for use in the increase of the M2/M1 macrophage ratio, prevention or treatment of inflammation, prevention or treatment of fibrosis of inflamed tissue, or suppression of adhesion molecule expression of blood vessels within inflamed tissue is provided.

According to the present disclosure, the extracellular vesicles of the present disclosure, or a composition (or pharmaceutical composition) comprising the said extracellular vesicles, for use in the increase of the M2/M1 macrophage ratio, prevention or treatment of inflammation, prevention or treatment of fibrosis of inflamed tissue, or suppression of adhesion molecule expression of blood vessels within inflamed tissue are provided.

According to the present disclosure, a method for treating a subject, comprising administering the extracellular vesicles of the present disclosure to the said subject, is provided. According to the present disclosure, a method for increasing the M2/M1 macrophage ratio, treating inflammation, treating fibrosis of inflamed tissue, or suppressing adhesion molecule expression of blood vessels within inflamed tissue in a subject in need thereof, comprising administering the extracellular vesicles of the present disclosure to the said subject, is provided. The subject may be a subject who has a possibility of developing inflammation in a tissue, a subject who has inflammation in a tissue, a subject who has fibrosis in a tissue, or a subject who has increased expression of vascular adhesion molecules.

**EXAMPLES**

Example 1: Promotion of Extracellular Vesicle Secretion and Preparation of Extracellular Vesicles by Culturing under Various Conditions

In this example, in addition to the method of obtaining extracellular vesicles by culturing mesenchymal stem cells in a normal medium, extracellular vesicles were obtained and analyzed by applying heat shock under various conditions to promote the secretion of extracellular vesicles.

Mesenchymal stem cells were cultured in a medium. Bone marrow-derived mesenchymal stem cells and umbilical cord-derived mesenchymal stem cells were used as the mesenchymal stem cells.

The cells were cultured at 37°C for 3 to 4 days. After culturing, the medium was replaced with a first medium, and heat shock was applied to the cells by culturing in the first medium (also referred to as starvation medium in the present specification) at 42°C for 1.5 hours. The first medium (starvation medium) was a serum-free and albumin-free DMEM medium, and did not comprise any of other growth factors, insulin, and transferrin. However, the first medium suitably maintained the morphology of the mesenchymal stem cells, the ability to differentiate into adipocytes, osteoblasts, and chondrocytes, the self-renewal ability, and the expression profiles of cell surface markers (particularly CD73, CD90, CD105, HLA-ABC). Cells under conditions where heat shock was not applied were cultured at 37°C for 1.5 hours. After heat shock, the medium was replaced with a second medium (also referred to as recovery medium in the present specification), or the cells were cultured in the first medium at 37°C for 48 to 96 hours. The conditions of each condition, the presence or absence of heat shock and the conditions of heat shock, and the subsequent culturing conditions were as shown in Table 1. As for the second medium, a serum-free and xeno-free medium using Dulbecco's Modified Eagle Medium (DMEM) as the basal medium was used. Factors typically added to the medium (4.5 g/L glucose, albumin, and cell proliferation factors) were added to the second medium for the purpose of promoting cell proliferation. Specifically, 1 to 5% human serum albumin (HSA) was added to the second medium. The second medium further comprised basic fibroblast growth factor (bFGF), transforming growth factor (TGF) β3, platelet-derived growth factor (PDGFbb), transferrin, and insulin. Subsequently, the supernatant of the cells treated under each condition was recovered.

[Table 1]

| Table 1: Culturing Conditions | | |
|---|---|---|
| Condition | Heat Shock | After Heat Shock |
| Condition 1 | None (in second medium) | Second medium |
| Condition 2 | Yes (in second medium) | Second medium |
| Condition 3 | None (in second medium) | First medium |
| Condition 4 | Yes (in first medium) | First medium |
| Condition 5 | Yes (in first medium) | Second medium |

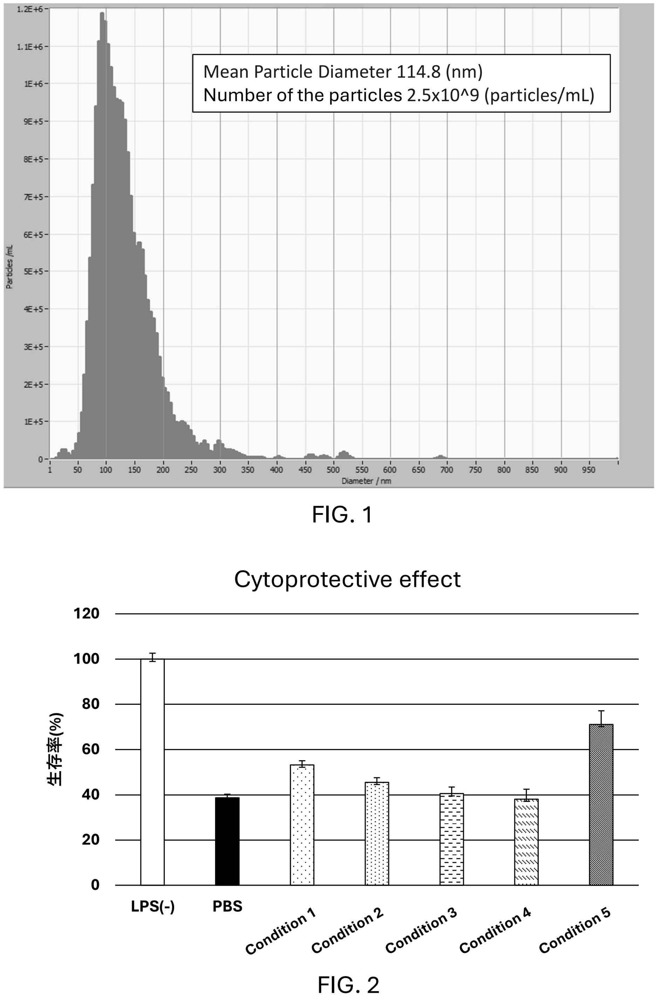

The recovered culture supernatant was centrifuged at 2,000×g for 10 minutes at 4°C to remove cell components and apoptotic bodies of about 1 μm, and then microvesicles were removed by filtering with a filter having a pore size of 0.22 μm. Then, extracellular vesicles (EVs) were concentrated by ultrafiltration. The obtained extracellular vesicles were further subjected to size exclusion chromatography to obtain an EV solution comprising exosomes. The purified EV solution was sterilized by filtering with a filter having a pore size of 0.22 μm. The particle size and particle number of the obtained EVs were measured using ZetaView (Particle Metrix). The EVs obtained by Condition 5 were diluted 50 to 500 times with phosphate buffered saline (PBS), and the particle size and particle number were measured in triplicate (see FIG. 1). Although some differences were observed depending on the condition, no significant difference was observed in the particle size distribution.

Next, the concentration of various markers in the EVs purified above was measured. The amount of CD81 in the EVs was quantified by ELISA according to the product protocol of the CD81/CD81 Exosome ELISA Kit (Hakarel Co., Ltd., HAK-HEL8181-1). The quantified amount of CD81 was corrected by the particle number of each sample, and the amount of CD81 per particle number was calculated. The amount of CD63 in the EVs was quantified by ELISA according to the product protocol of the Human CD63 ELISA Kit (abcam, ab275099). CD81 is considered to be a marker for exosomes, and marker expression indicates that exosomes are included in the EVs obtained.

The miRNA in the EVs was extracted according to the product protocol using the MicroRNA Extractor (trademark) SP Kit (Fujifilm Wako Pure Chemical Corporation, 295-71701). The EVs were appropriately diluted with a solvent so that the particle numbers between the comparative samples were equal. The extracted miRNA was quantified by qPCR using a common primer attached to the product and the primers shown in Table 2 after reverse transcription according to the product protocol using the Mir-X miRNA qRT-PCR TB Green Kit (Takara Bio Inc., 638314). In addition, for each miRNA, a mimic with a known concentration was used to perform the same reverse transcription reaction as the miRNA sample, and serial dilutions were used as the standard. The standard was also subjected to qPCR in the same manner as the miRNA sample, and the concentration of miRNA was quantified using the standard curve created thereby. The miRNA content per particle was calculated from the quantified miRNA concentration and the EV particle number used during extraction.

[Table 2]

| Table 2: Sequences of miRNA-Specific Primers Used | |
|---|---|
| miRNA | Sequence |
| miR-4516 | GGGAGAAGGGUCGGGGC |
| miR-324-5p | CGCATCCCCTAGGGCATTGGTG |

The results were as shown in Table 3.

[Table 3]

| Table 3: CD81 Expression and miRNA Expression | | |
|---|---|---|
| | CD81 Expression | miRNA *2 |
| Condition | CD81 per Particle *1 | miR-4516 |
| 1 | 0.98 | 1,700 |
| 2 | 1.01 | 2,200 |
| 3 | 0.59 | 64,000 |

(continued)

| Table 3: CD81 Expression and miRNA Expression | | |
|---|---|---|
| | CD81 Expression | miRNA *2 |
| Condition | CD81 per Particle *1 | miR-4516 |
| 4 | 0.43 | 47,000 |
| 5 | 1.00 | 3,900 |
| *1: Numerical value is relative ratio to Condition 5<br>*2: Unit is amol/$10^9$ particles | | |

Condition 1 is the conventional standard method. Among Conditions 2 to 4, only in Condition 5 was the CD81 content equivalent to that of Condition 1, and the content of the said miR-4516 was higher than that of Condition 1. Condition 2 showed almost no increase in the content of the said miR-4516 of miRNA, and Conditions 3 and 4 showed low CD81 expression. "amol" means attomole, i.e., $10^{-18}$ mol. "ag" means attogram, i.e., $10^{-18}$ g. From the viewpoint of quality control of extracellular vesicles, a higher content ratio of CD81-positive exosomes is considered preferable. Conditions 3 and 4 also showed a certain CD81-positive exosome content ratio and are preferable, but Conditions 1, 2, and 5 showed a higher CD81-positive exosome content ratio and are more preferable. Among Conditions 1, 2, and 5, Condition 5 achieved both a high CD81-positive exosome content ratio and miR-4516 concentration, and is the most preferable from the viewpoint of CD81-based quality control.

From the above results, Condition 5 was considered suitable for increasing the ratio of CD81-positive exosomes per particle and/or increasing the concentration of miR-4516 in the exosomes.

Furthermore, to quantify the CD81 amount more accurately, the amount of CD81 in the EVs was quantified by LC-MS/MS. Specifically, the High Performance Liquid Chromatograph was Ultimate 3000 (Thermo Fisher Scientific), the Mass Spectrometer was TSQ-Quantiva (Thermo Fisher Scientific), and the Ion Source was H-ESI (Thermo Fisher Scientific). A CD81 peptide was synthesized, a standard sample having 7 concentration points in the concentration range of 10 fmol/$\mu$L to 250 fmol/$\mu$L was prepared, and MRM (SRM) LC-MS/MS proteome analysis was performed by the internal standard method. As a result, the CD81 contained in the EVs was 0.13 $\mu$g/$1 \times 10^9$ particles, which is 0.13 fg/particle (130 ag/particle). As a reference, when the amount of CD81 under condition 3 is calculated based on the ratio in Table 3, it is estimated to be approximately 77 ag per particle. This value was consistent with the value measured by ELISA (27 ag/particle in Condition 5). The fact that the numerical value changes depending on the measurement system is not a problem itself, but considering the error due to the measurement system, it is desirable to specify CD81 by the ratio to the CD81 expression amount when heat shock is not performed.

The expression of miR-324-5p was 0.22 amol/$10^9$ particles in Condition 1, 0.59 amol/$10^9$ particles in Condition 2, 0.64 amol/$10^9$ particles in Condition 3, 1.3 amol/$10^9$ particles in Condition 4, and 0.89 amol/$10^9$ particles in Condition 4. This suggested the possibility that Condition 5 also increases the concentration of anti-inflammatory factors other than miR-4516 in exosomes.

Condition 5 involves applying heat shock to cells in a starvation medium, which is the simplest condition for applying heat shock stress. It is possible that this increased the anti-inflammatory property of the released exosomes, and subsequently culturing the cells in a normal medium for recovery improved the quality of the exosomes released from the treated cells. The second medium after heat shock only needs to contain nutrients (for example, glucose and L-glutamine, and preferably albumin as well) that exceed a poor nutrient medium, which promotes the production of exosomes and their contents based on the signal from heat shock stress, and thus enables the recovery of anti-inflammatory exosomes. Furthermore, by including nutrients and preferably cell proliferation factors (bFGF, TGF$\beta$, PDGF, etc.) in the second medium to promote cell proliferation, the production of exosomes and their contents based on the signal from heat shock stress is further promoted. A person skilled in the art will be able to appropriately design and use a medium composition that activates cell proliferation. The reason why CD81 expression decreases due to heat shock is unknown. However, since CD81 is an exosome marker, the decrease in CD81 suggests either an increase in CD81-negative exosomes caused by heat shock or a decrease in exosome purity due to an increase in cell membrane fragments or apoptotic bodies. Culturing the cells in the second medium may have increased the number of exosomes derived from healthy cells.

The miRNA expression amount may fluctuate when the type of starting cell is changed, but the expression ratios of Conditions 1 to 5 were generally maintained in all tested cell types (bone marrow-derived and umbilical cord-derived).

Example 2: Inhibitory Effect on Silica-Induced Cell Death in Alveolar Epithelial Cells

Pulmonary fibrosis is a pathological condition in which excessive accumulation of extracellular matrix in the lung causes destruction of alveolar structure, thickening of the alveolar wall, and leads to respiratory failure. Some pulmonary fibrosis is caused by the inhalation of fine particles such as silica and asbestos. Therefore, to evaluate the effect of EXP01 on injury to alveolar epithelial cells that induces fibrosis, A549 cells were seeded in a culture plate, and silica (SIGMA) was added to induce cell death. Then, EVs prepared by each manufacturing method or PBS were added 1 hour before the induction of cell death, and the cell number after 48 hours was measured. The cell number was measured by the absorbance of the chemical substance WST-8, which changes its wavelength in living cells, using the commercially available assay kit Cell Counting Kit-8 (Dojindo Laboratories). The evaluation was performed by calculating the cell viability rate of each condition, assuming that the absorbance under the non-silica addition condition means 100% viable cells. The results were as shown in FIG. 2. As shown in FIG. 2, the EVs obtained by Condition 5 significantly suppressed silica-induced cell death.

Example 3: Anti-inflammatory Effect of EVs

Example 3-1: Effect on M1/M2 Macrophage Differentiation

M1 macrophages are immune cells that are responsible for defense against pathogens such as bacteria and viruses and promote an inflammatory response. In contrast, M2 macrophages are immune cells that support the resolution of inflammation and tissue repair. M1 macrophages and M2 macrophages can differentiate from M0 monocytes to M1 or from M0 to M2 depending on their environment, and a decrease in M1 and an increase in M2 mean the suppression of tissue inflammation.

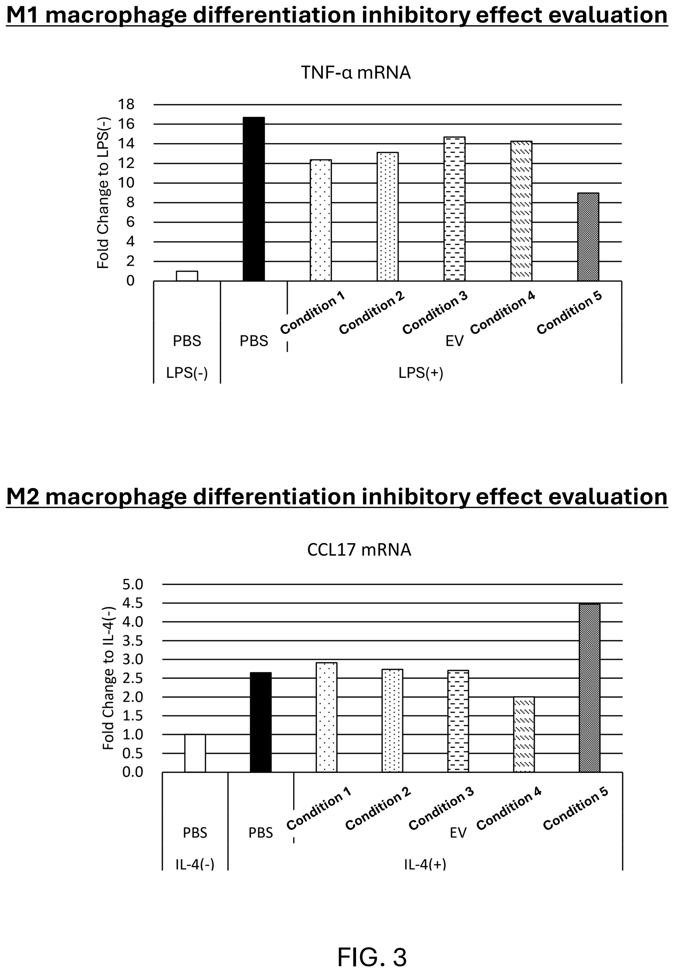

THP-1 (human monocyte-derived cell line) was differentiated into M0 macrophages by adding phorbol 12-myristate 13-acetate (PMA), and then a part was differentiated into M1 macrophages by adding lipopolysaccharide (LPS), and another part was differentiated into M2 macrophages by adding interleukin (IL)-4. EVs were added to the cells 1 hour or 24 hours before the induction of differentiation, and the mRNA expression of each differentiation marker (tumor necrosis factor (TNF)-$\alpha$, which is an M1 marker, and CCL17, which is an M2 marker) after 24 or 48 hours was analyzed by quantitative RT-PCR. The expression amount was calculated as a relative value with the expression amount in M0 as 1, using the $\Delta\Delta$Ct method for the analysis. The results were as shown in FIG. 3. As shown in FIG. 3, the EVs obtained by Condition 5 suppressed the increase of M1 macrophages induced by LPS and promoted the increase of M2 macrophages. This suggested that the EVs obtained by Condition 5 induce differentiation toward M2.

Example 3-2: Anti-inflammatory Effect in a Pneumonia Model

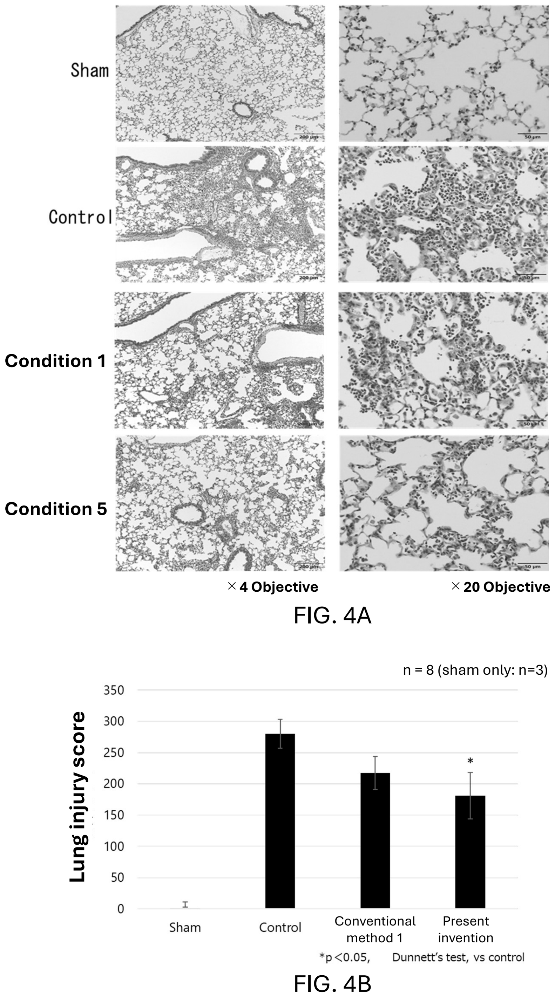

In this example, the anti-inflammatory effect was confirmed by administering EVs to a mouse model presenting Acute Respiratory Distress Syndrome (ARDS) as a pneumonia model. Specifically, to evaluate the effect of EVs on acute lung injury due to infection, C57BL/6J mice were administered Dulbecco's Phosphate Buffered Saline (non-inflammation-elicited group: Sham) or E. coli-derived LPS (inflammation-elicited group) at 100 $\mu$g/head intratracheally to create a lung injury mouse model. PBS (Control group) or EVs were administered intravenously at 0.1 mL/head 1 hour after the induction of inflammation. Bronchoalveolar lavage fluid was recovered after 72 hours, and then 10% neutral buffered formalin solution was injected into the lung to prepare tissue sections of the right lower lobe of the lung stained with hematoxylin and eosin. A histopathological examination was performed using a microscope. Photographs of each group were taken (low-power view and high-power view). For the low-power view, one field of view was imaged at ×4 objective magnification, and for the high-power view, 20 fields of view were imaged at ×40 objective magnification. Representative photographs are shown in FIG. 4A. The photographs were taken randomly, except for fields containing 50% or more alveoli and fields containing a mixture of the lumen of the trachea and blood vessels. The evaluation was performed according to the lung injury score shown in Table 4 below (Am J Respir Cell Mol Biol, 2011: 44: 725-737).

Table 4: Lung Injury Score

| Parameter/Score | 0 points | 1 point | 2 points |
|---|---|---|---|
| A. Neutrophils in alveolar space | None | 1 to 5 | > 5 |
| B. Neutrophils in interstitium | None | 1 to 5 | > 5 |
| C. Hyaline membranes | None | 1 | > 1 |
| D. Proteinaceous material | None | 1 | > 1 |
| E. Alveolar septal thickening | < 2 times | 2 to 4 times | > 4 times |

The lung injury score was calculated from the observed results by the following formula.

$$\text{Score (points)} = [(20 \times A) + (14 \times B) + (7 \times C) + (7 \times D) + (2 \times E)]/(\text{Number of fields} \times 100)$$

The scoring results are shown in FIG. 4B. As shown in FIG. 4B, a significant decrease in the lung injury score was observed in Condition 5.

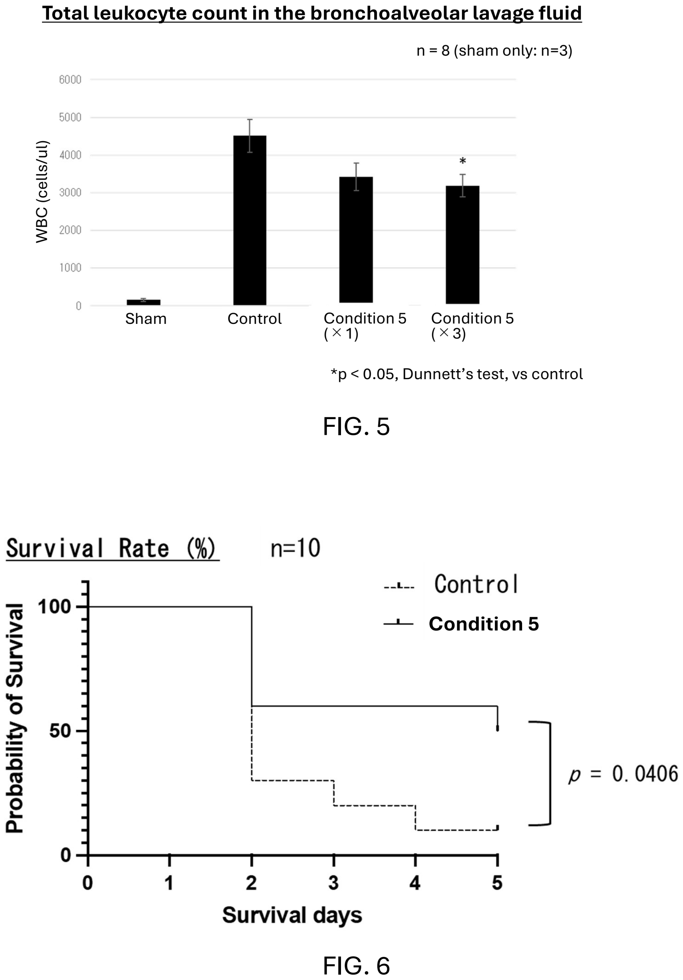

The leukocyte count in the bronchoalveolar lavage fluid recovered above was measured using a multi-parameter automated hematology analyzer (XT-2000iV, Sysmex Corporation). The results were as shown in FIG. 5. As shown in FIG. 5, the EVs obtained by Condition 5 significantly reduced the total leukocyte count in the bronchoalveolar lavage fluid, which also suggested a strong anti-inflammatory effect.

Example 3-3: Efficacy in ARDS Sepsis Model

To evaluate the effect of EVs on sepsis, C57BL/6J mice were administered *E. coli* (O111:B4)-derived LPS (15 mg/kg) intraperitoneally to create a sepsis model. PBS (Control group) or EVs were administered intravenously at 0.1 mL/head 1 hour after LPS administration. The mice were observed for 5 days, and the survival rate was evaluated. The results were as shown in FIG. 6. As shown in FIG. 6, the EVs obtained by Condition 5 significantly improved the survival rate of the sepsis model.

Example 4: Inhibitory Effect on Adhesion Molecule Expression in Vascular Endothelial Cells

Adhesion molecules produced by vascular endothelial cells are involved in the initial reaction of inflammatory cell infiltration into inflamed tissue such as lung tissue in ARDS. For example, an increase in blood adhesion molecules has been reported in ARDS patients. Therefore, the pharmacological effect of EVs on adhesion molecule production in vascular endothelial cells was evaluated using the human immortalized umbilical vein endothelial cell line HUEhT-1.

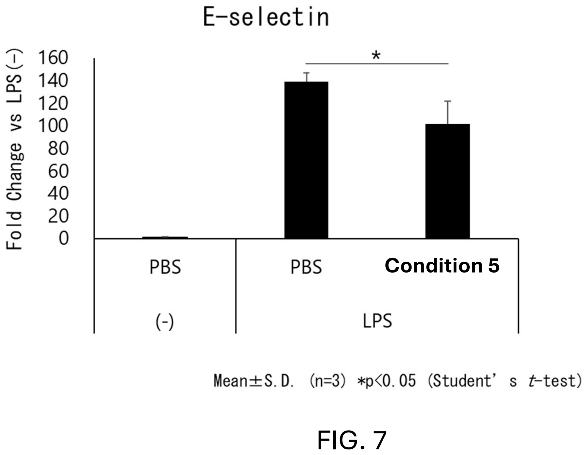

In the test, EVs were added to HUEhT-1 cells, and inflammation was induced by adding LPS after 48 hours. The mRNA expression of E-selectin 6 hours later was analyzed using the quantitative RT-PCR method. The analysis was performed using the $\Delta\Delta$Ct method, and evaluated as a relative value with the expression amount in the non-stimulated control as 1. The results were as shown in FIG. 7. As shown in FIG. 7, the EVs obtained by Condition 5 significantly inhibited the expression of E-selectin induced by LPS.

**Claims**

1. An aqueous composition comprising extracellular vesicles, wherein:
   the extracellular vesicles are:

   (i) expressing CD81, the expression amount of CD81 per extracellular vesicle is greater than or equal to a first reference value, and the first reference value is a value of 80 (ag/particle) or more, or is greater than or equal to a first reference magnification relative to the expression amount of CD81 in extracellular vesicles obtained by the same separation method from the same cell population under conditions (for example, in a second medium) where heat shock treatment is not performed and the treatment of changing the type of medium is not performed, and the first reference magnification is a magnification of 0.6 times or more, 0.7 times or more, 0.8 times or more, 0.9 times or more, or 1 time or more (for example, 0.6 times to 1.1 times, for example, 0.8 times to 1.05 times, or 0.9 times to 1.1 times), and,
   (ii) the extracellular vesicles satisfy one or more selected from the group consisting of the following (ii-1) to (ii-2):

   (ii-1) the extracellular vesicles express miR-4516, the expression amount thereof is greater than or equal to a second reference value, and the second reference value is a value of 500 amol/$10^9$ particles or more, and
   (ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more.

2. The aqueous composition according to Claim 1, wherein
   (ii-1) the extracellular vesicles express miR-4516, the expression amount thereof is greater than or equal to a second reference value, and the second reference value is a value of 2500 amol/$10^9$ particles or more.

3. The aqueous composition according to Claim 1, wherein:

(ii-2) the expression amount of miR-4516 is a value greater than or equal to a second reference magnification relative to the expression amount of miR-4516 in extracellular vesicles obtained by the same separation method from the same cell population under conditions (for example, in a second medium) where heat shock treatment is not performed and the treatment of changing the type of medium is not performed, and the second reference magnification is a value of 1.3 times or more.

4. The aqueous composition according to Claim 1, wherein the extracellular vesicles satisfy (ii-1) and (ii-2).

5. A lyophilized product of the composition according to any one of Claims 1 to 4.

6. A kit for extemporaneous preparation of an extracellular vesicle preparation, comprising the lyophilized product according to Claim 5 and water for reconstitution.

7. A method for manufacturing an aqueous composition comprising extracellular vesicles, comprising:

culturing mesenchymal stem cells under normal nutrient conditions;
subsequently, inducing a heat shock response in the said mesenchymal stem cells under serum-free, albumin-free, platelet lysate-free, and growth factor-free conditions;
further subsequently, culturing mesenchymal stem cells in a medium that does not contain foreign exosomes and is suitable for cell proliferation, thereby secreting extracellular vesicles into the supernatant of the said medium, and recovering the supernatant comprising extracellular vesicles; and
separating the extracellular vesicles from the said supernatant to obtain an aqueous composition comprising extracellular vesicles.

8. The method according to Claim 7, further comprising filter sterilization.

9. The composition according to any one of Claims 1 to 4, the lyophilized product according to Claim 5, or the kit according to Claim 6, obtainable by the method according to Claim 7 or 8.

10. The composition according to any one of Claims 1 to 4, the lyophilized product according to Claim 5, or the kit according to Claim 6, which is a pharmaceutical composition for use in treating inflammation in a subject.

11. The composition, lyophilized product, or kit according to Claim 9, wherein the inflammation is pneumonia.

12. The composition, lyophilized product, or kit according to Claim 10, wherein the inflammation is systemic inflammation.

13. The composition, lyophilized product, or kit according to Claim 10 or 12, wherein the inflammation is sepsis.

14. The composition according to any one of Claims 1 to 4, the lyophilized product according to Claim 5, or the kit according to Claim 6, for use in suppressing the expression of an adhesion molecule of vascular endothelial cells in a subject.

15. The composition, lyophilized product, or kit according to Claim 14, wherein the adhesion molecule is E-selectin.

FIG. 1

Cytoprotective effect

FIG. 2

## M1 macrophage differentiation inhibitory effect evaluation

**TNF-α mRNA**

## M2 macrophage differentiation inhibitory effect evaluation

**CCL17 mRNA**

FIG. 3

× 4 Objective                    × 20 Objective

FIG. 4A

n = 8 (sham only: n=3)

*p < 0.05,    Dunnett's test, vs control

FIG. 4B

**Total leukocyte count in the bronchoalveolar lavage fluid**

n = 8 (sham only: n=3)

*p < 0.05, Dunnett's test, vs control

FIG. 5

FIG. 6

FIG. 7

## INTERNATIONAL SEARCH REPORT

| | International application No. |
|---|---|
| | **PCT/JP2024/018805** |

| A. | CLASSIFICATION OF SUBJECT MATTER |
|---|---|

*C12N 5/0775*(2010.01)i; *A61K 9/19*(2006.01)i; *A61K 35/12*(2015.01)i; *A61P 11/00*(2006.01)i; *A61P 29/00*(2006.01)i; *A61P 31/04*(2006.01)i; *C12N 1/00*(2006.01)i; *C12N 15/113*(2010.01)i; *C12P 1/00*(2006.01)i

FI: C12N5/0775; C12N1/00 N; C12P1/00 Z; C12N1/00 A; C12N1/00 K; A61K9/19; A61K35/12; A61P11/00; A61P29/00; A61P31/04; C12N15/113 Z ZNA

According to International Patent Classification (IPC) or to both national classification and IPC

| B. | FIELDS SEARCHED |
|---|---|

Minimum documentation searched (classification system followed by classification symbols)

C12N5/0775; A61K9/19; A61K35/12; A61P11/00; A61P29/00; A61P31/04; C12N1/00; C12N15/113; C12P1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 西部隆宏, 第3回　細胞外小胞を利用した治療の実現をサポートする富士フイルム和光純薬の取り組み, 和光純薬時報, 15 January 2023, vol. 91, no. 1, pp. 16-18, http://fujifilm.com/ffwk, (NISHIBU, Takahiro. Wako Pure Chemical Times.), non-official translation (Part 3: Fujifilm Wako Pure Chemical's efforts to support the realization of treatment using extracellular vesicles) pp. 16, 17 | 1, 5, 6, 9-15 |
| A | | 2-4, 7 |
| X | JP 2022-544698 A (BREXOGEN INC.) 20 October 2022 (2022-10-20) pp. 15, 16 | 1, 5, 6, 9-15 |
| A | | 2-4, 7 |
| X | WO 2022/025007 A1 (EXORPHIA INC.) 03 February 2022 (2022-02-03) example 1 | 1, 5, 6, 9-15 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **31 July 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/018805**

### C. DOCUMENTS CONSIDERED TO BE RELEVANT

| Category* | Citation of document, with indication, where appropriate, of the relevant passages | Relevant to claim No. |
|---|---|---|
| A | YUCE, M. ALBAYRAK, E. Hyperthermia-stimulated tonsil-mesenchymal stromal cells suppress hematological cancer cells through downregulation of IL-6. Journal of Cellular Biochemistry. 2022, vol. 123, pp. 1966-1979, doi:10.1002/jcb.30322 <br> entire text, all drawings | 1-15 |

Form PCT/ISA/210 (second sheet) (July 2022)

## INTERNATIONAL SEARCH REPORT
### Information on patent family members

International application No.

**PCT/JP2024/018805**

| Patent document cited in search report | | | Publication date (day/month/year) | Patent family member(s) | | | Publication date (day/month/year) |
|---|---|---|---|---|---|---|---|
| JP | 2022-544698 | A | 20 October 2022 | US | 2022/0233601 | A1 | |
| | | | | pp. 7, 8 | | | |
| | | | | WO | 2021/033990 | A1 | |
| | | | | EP | 4019027 | A1 | |
| | | | | KR | 10-2021-0024417 | A | |
| | | | | CN | 114025776 | A | |
| WO | 2022/025007 | A1 | 03 February 2022 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. YUCE ; E. ALBAYRAK**. *Journal of Cellular Biochemistry*, 2022, vol. 132 (2), 1966-1979
- **T. YAND et al.** *Journal of Biological Engineering*, 2022, vol. 16 (24)
- **R. KORE et al.** *American Journal of Physiology-Regulatory, Integrative and Comparative Physiology*, 2021, vol. 321 (5), R639-R654
- **L. WANG et al.** *Analytical Cellular Pathology*, 2022 (8708202)
- **YOON et al.** *CELLs*, 2021, vol. 10 (7), 1682
- **CHOWDHARI et al.** *Biochim Biophys Acta Mol Basis Dis*, 2017, vol. 1863 (12), 3142-3152
- **PAO et al.** *PloS One*, 2022, vol. 17 (6), e0270526
- **XU et al.** *Mol. Ther.*, 2022, vol. 30 (6), 2370-2387
- *Am J Respir Cell Mol Biol*, 2011, vol. 44, 725-737